# EUROPEAN PATENT APPLICATION

(11) **EP 4 147 689 A1**
(43) Date of publication of application: **15.03.2023**
(21) Application number: 21196272.5
(22) Date of filing: 13.09.2021
(51) Int. Cl.: A61K 9/16, A61K 9/20, A61K 31/00, A61P 1/00

(54) **LENVATINIB FORMULATION**

(71) Applicant: Lotus Pharmaceutical Co., Ltd., 11046 Taipei City (TW)
(72) Inventor: Mehta, Gaurav Bhupendrabhai, Nantou City (TW); Gupta, Vijender, Nantou City (TW); Chawla, Manish, Nantou City (TW)
(74) Representative: Hartvichova, Katerina

(57) **Abstract**

The present invention provides a pharmaceutical formulation comprising Lenvatinib or a pharmaceutically acceptable salt thereof and at least one alkalizer, and optionally at least one disintegrant. The formulation is stable and has a beneficial dissolution profile.

## Description

### Field of Art

The present invention relates to pharmaceutical formulations of lenvatinib.

### Background Art

Lenvatinib is a pharmaceutically active ingredient which is used as a medicament for the treatment of cancers, in particular thyroid cancers, renal cell carcinoma (in combination with everolimus), hepatocellular carcinoma, endometrial carcinoma (in combination with pembrolizumab). It is sold under the brand name Lenvima and also under other brand names. Lenvatinib acts as a VEGFR1, VEGFR2 and VEGFR3 kinase inhibitor.

Lenvatinib was first disclosed in EP1415987, together with pharmaceutically acceptable salts thereof.

However, lenvatinib in pharmaceutical formulations easily decomposes under humid and warm storage conditions; and moreover, gelation readily occurs on the surface of the pharmaceutical compositions, so that when the pharmaceutical compositions are stored under humid conditions, delayed dissolution of the active ingredient occurs due to moisture absorption.

EP1797881 discloses a pharmaceutical composition that solves the above problems using an alkaline excipient with a pH of 8 (in 5 % w/w aqueous solution) to reduce the degradation of the active substance and silicic acid to inhibit gelation. Sodium carbonates are described as suitable alkaline excipient. EP2468281 discloses that when an alkaline earth metal carbonate is used as a base in combination with a disintegrant, the pharmaceutical compositions have superior dissolution properties in comparison with compositions containing bases, even after long term storage in HPMC capsule shell. During prosecution the applicant provided results showing that when other carbonates and bicarbonates such as sodium bicarbonate were used as stabilizers, the dissolution rate decreased after storage as compared to before storage and the dissolution time was additionally delayed.

EP3384901 discloses that the usage of sodium carbonate along with the Lenvatinib mesylate shows better dissolution.

There is a continuous need to design Lenvatinib formulations which would overcome the above drawbacks.

### Disclosure of the Invention

The present invention provides a pharmaceutical formulation which comprises Lenvatinib or a pharmaceutically acceptable salt thereof and at least one alkalizer.

In a preferred embodiment, the formulation further comprises at least one disintegrant. Yet more preferably, the formulation further comprises at least one binder.

In some embodiments, the weight ratio of Lenvatinib or a pharmaceutically acceptable salt thereof to the at least one alkalizer is from 1:0.3 to 1:6, preferably 1:1 to 1:4.

In some embodiments, the weight ratio of Lenvatinib or a pharmaceutically acceptable salt thereof to the at least one disintegrant is 1:1 to 1:6, preferably 1:1 to 1:3.

In some embodiments, the pharmaceutical formulation contains from 10 to 20 wt. % of Lenvatinib or a pharmaceutically acceptable salt thereof, from 4 to 50 wt. % of at least one alkalizer, and optionally also 20 to 40 wt. % of at least one disintegrant. The wt. % are relative to the weight of the pharmaceutical formulation granulate, i.e., not taking into account the weight of a capsule or of a tablet coating.

More preferably, the pharmaceutical formulation contains from 10 to 15 wt. % of Lenvatinib or a pharmaceutically acceptable salt thereof.

More preferably, the pharmaceutical formulation contains from 15 to 50 wt. %, even more preferably from 30 to 40 wt. %, of at least one alkalizer, and yet more preferably from 35 to 42 wt. % of at least one alkalizer.

More preferably, the pharmaceutical formulation contains from 20 to 30 wt. %, even more preferably from 23 to 28 wt. % of at least one disintegrant.

More preferably, the pharmaceutical formulation contains up to 5 wt. %, even more preferably from 1 to 4 wt. % of at least one binder.

The pharmaceutical formulation may contain further pharmaceutically acceptable excipients such as diluent(s), binder(s), lubricant(s), colouring agent(s), flavouring agent(s), gelation inhibitor(s).

The pharmaceutical formulation is preferably further formulated into gelatine capsules, HPMC capsules, tablets or coated tablets.

Lenvatinib is a compound of the following formula:

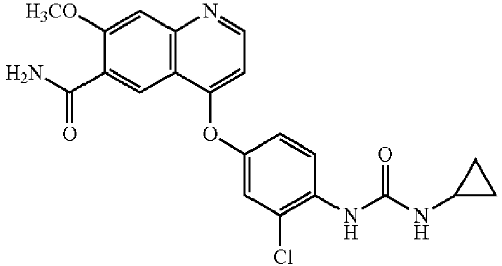

Pharmaceutically acceptable salts of lenvatinib are salts of organic and inorganic acids, such as acetate, aspartate, benzoate, chlorobenzoate, methylbenzoate, dinitrobenzoate, besylate, bicarbonate, carbonate, bisulfate, sulfate, pyrosulfate, bisulfite, sulfite, borate, camsylate, caprylate, citrate, edisylate, esylate, formate, fumarate, gluceptate, gluconate, glucuronate, hexafluorophosphate, hibenzate, hydrochloride, chloride, hydrobromide, bromide, hydroiodide, iodide, isethionate, isobutyrate, lactate, malate, maleate, malonate, mandelate, mesylate, methylsulfate, naphthylate, 2-napsylate, nicotinate, nitrate, orotate, oxalate, palmitate, pamoate, phosphate, hydrogen phosphate, dihydrogen phosphate, metaphosphate, pyrophosphate, phthalate, propionate, saccharate, sebacate, stearate, suberate, succinate, tartrate, tosylate, trifluoroacetate.

An especially preferred pharmaceutically acceptable salt of lenvatinib is lenvatinib mesylate.

Lenvatinib or a pharmaceutically acceptable salt thereof is preferably micronized, i.e., has a particle size D₉₀ (i.e., 90 % of the particles are within said range) ranging from 1 micron to 60 microns, most preferably D₉₀ within the range of 1 micron to 50 microns.

The alkalizers can be selected from the group consisting of magnesium oxide, calcium oxide, calcium chloride, sodium carbonate, lithium carbonate, potassium carbonate, sodium hydrogen carbonate, lithium hydrogen carbonate, potassium hydrogen carbonate, disodium hydrogenphosphate, sodium citrate, dipotassium hydrogenphosphate, sodium acetate, sodium lauryl sulphate, polacriline potassium, calcium hydroxide, sodium hydroxide, potassium hydroxide, meglumine, magnesium aluminometasilicate, mesoporous silica, colloidal silica and sodium hydroxide, with the proviso that sodium carbonate is only used in combination with at least one other alkalizer.

More preferably, the alkalizer is selected from potassium hydrogen carbonate, potassium carbonate, meglumine, sodium carbonate, sodium hydrogen carbonate, polacrilin potassium, magnesium aluminometasilicate, mesoporous silica, colloidal silica and magnesium oxide, with the proviso that sodium carbonate is only used in combination with at least one other alkalizer.

Most preferably, the alkalizer is selected from potassium hydrogen carbonate, meglumine, mixutre of potassium hydrogen carbonate with meglumine, and mixtures thereof with sodium carbonate.

Disintegrants can be selected from corn starch, partially pregelatinized starch, hydroxypropyl starch, carmellose, carmellose sodium, carmellose calcium, carboxymethyl starch sodium, croscarmellose sodium, low-substituted hydroxypropylcellulose and crospovidone.

Disintegrant is preferably selected from the group comprising low-substituted hydroxypropyl cellulose (L-HPC) and croscarmellose sodium.

Diluents can be selected from the group consisting of lactose, sucrose, glucose, corn starch, mannitol, sorbitol, starch, alpha starch, dextrin, magnesium aluminometasilicate.

Binders can be selected from the group consisting of hydroxypropyl cellulose, gelatin, hydroxy propyl methylcellulose, sodium carboxymethylcellulose, polyvinylpyrrolidone, Macrogol, microcrystalline cellulose. Especially preferred binder is hydroxypropyl cellulose.

Lubricants can be selected from the group consisting of magnesium stearate, calcium stearate, sodium stearyl fumarate, talc, colloidal silica.

Gelation inhibitor can be selected from the group consisting of light anhydrous silicic acid, silicon dioxide hydrate, calcium silicate, magnesium silicate, magnesium aluminosilicate, magnesium aluminium silicate, synthetic aluminium silicate, calcium chloride, calcium carbonate, magnesium carbonate and hydrous silicic dioxide.

The pharmaceutical formulation of the invention stabilizes the composition, prevents problems with dissolution, and prevents the surface gelation.

Meglumine and mixtures of meglumine with other alkalizers result in especially beneficial dissolution properties.

In addition to this, potassium carbonate and potassium hydrogen carbonate produce, upon reaction with hydrochloric acid in the stomach, potassium chloride, water and carbon dioxide, which causes the active ingredient to disperse in very fine particles, which further helps to avoid gelation.

Yet furthermore, potassium carbonate and potassium hydrogen carbonate have a better solubility in water than alkaline earth metal carbonates, thus improving the pharmaceutical processability, in particular in wet granulation with water or in wet granulation with ethanol or in dry granulation process or in roller compaction process.

Gelatine capsule shells provide a better dissolution rate than other final formulations, due to improved prevention of surface gelation.

The formulations of the present invention are preferably prepared using wet granulation process or spray granulation process.

The formulations of the present invention are for use as a medicament, in particular for the treatment of cancers, in particular thyroid cancers, renal cell carcinoma, hepatocellular carcinoma or endometrial carcinoma.

### Brief Description of Drawings

Figure 1 is a graph showing the dissolution profile of capsules comprising formulations according to the invention (Example 3, Example 10) in comparison with Lenvima^{(R)} and with examples from EP3 384901.
Figure 2 is a graph showing the dissolution profile of granular formulations according to the invention (Example 3, Example 10) in comparison with Lenvima^{(R)} and with examples from EP3384901.
Figure 3 is a graph showing the dissolution profile of formulations according to the invention with different alkalizers, in comparison with Lenvima^{(R)}.

### Examples

### Examples 1-4, 1A-4A: Formulations comprising potassium hydrogen carbonate as alkalizer

The formulations were prepared using wet granulation process. The compositions of the formulations as filled into HPMC capsules are shown in the below Table 1 for Examples 1-4. Examples 1A-4A refer to these formulations, filled into gelatin capsules.

Wet granulation was performed with purified water as a solvent using a high-shear granulator with the polymorph C form crystal of 4-(3-chloro-4-(cyclopropylaminocarbonyl)aminophenoxy)-7-methoxy-6-quinolinecarboxamide methanesulfonate, mannitol 25C (parlitol) (Mannitol, Roquette), potassium hydrogen carbonate (Merck), hydroxypropyl cellulose (HPGL, Nippon Soda), low-substituted hydroxypropylcellulose (L-HPC (LH-11), Shin-Etsu Chemical) and microcrystalline cellulose (FMC) according to the formulation proportions in Table 1. The granules of which a moisture content was reduced to be less than 2% by further drying were sized using a screen mill, so that their granule diameters were less than 1 mm. Then, microcrystalline cellulose and talc were added to the sized granules according to the formulation proportions in Table 1, and the mixture was thoroughly mixed using a diffusion mixer. Hard HPMC capsules size 4 (Table 1) or hard gelatine capsules size 3 (Table 2) were filled with 100 mg of the resultant granules to prepare capsules containing the Lenvatinib mesylate.

**Table 1**

| Component | Example 1 | Exapmple 2 | Example 3 | Example 4 |
|---|---|---|---|---|
| | mg/capsule | mg/capsule | mg/capsule | mg/capsule |
| Lenvatinib Mesylate | 12.25 | 12.25 | 12.25 | 12.25 |
| Mannitol 25 C | 9.75 | 9.75 | 9.75 | 9.75 |
| Potassium hydrogen carbonate | 4.00 | 15.00 | 37.00 | 47.00 |
| Hydroxypropyl cellulose | 3.00 | 3.00 | 3.00 | 3.00 |
| Low substitued hydroxypropyl cellulose | 25.00 | 25.00 | 25.00 | 25.00 |
| Microcrystalline cellulose | 43.00 | 32.00 | 10.00 | 0.00 |
| Talc | 3.00 | 3.00 | 3.00 | 3.00 |
| Total weight of granules | 100.00 | 100.00 | 100.00 | 100.00 |
| HPMC capsules size 4 | 38.00 | 38.00 | 38.00 | 38.00 |
| Total weight of filled capsules | 138.00 | 138.00 | 138.00 | 138.00 |

**Table 2**

| Component | Example 1A | Example 2A | Example 3A | Example 4A |
|---|---|---|---|---|
| | mg/capsule | mg/capsule | mg/capsule | mg/capsule |
| Granules | 100.00 (Ex.1) | 100.00 (Ex.2) | 100.00 (Ex.3) | 100.00 (Ex.4) |
| Hard gelatin capsule shell size 3 | 47.00 | 47.00 | 47.00 | 47.00 |
| Total weight of filled capsules | 147.00 | 147.00 | 147.00 | 147.00 |

### Examples 5-8, 5A-8A: Formulations comprising potassium hydrogen carbonate as alkalizer

Examples 5 and 5A are comparative examples.

The formulations were prepared using top spray granulation tablet process. The compositions of the formulations as pressed into tablets are shown in the below Table 3 for Examples 5-8. Examples 5A-8A refer to these tablets, filled into gelatin capsules (Table 4).

Spray granulation was performed with Ethanol as a solvent with lenvatinib mesylate drug substance with mannitol 25C (parlitol) (Mannitol, Roquette), potassium hydrogen carbonate (Merck), hydroxypropyl cellulose (HPGL, Nippon Soda), low-substituted hydroxypropylcellulose (L-HPC (LH-11), Shin-Etsu Chemical) and microcrystalline cellulose (FMC) according to the formulation proportions in Table 3. The granules of which a moisture content was reduced to be less than 2% by further drying were sized using a screen mill, so that their granule diameters were less than 1 mm. Then, microcrystalline cellulose and magnesium stearate (Roquette) were added to the sized granules according to the formulation proportions in Table 3, and the mixture was thoroughly mixed using a diffusion mixer. Further the granules are compressed by using the 12 mm round punches and further coated by using Opadry yellow (Opadry contains hypromellose, iron oxide yellow and macrogol).

**Table 3**

| Component | Example 5 | Example 6 | Example 7 | Example 8 |
|---|---|---|---|---|
| | mg/tablet | mg/tablet | mg/tablet | mg/tablet |
| Lenvatinib mesylate | 12.25 | 12.25 | 12.25 | 12.25 |
| Mannitol 25 C | 5.75 | 4.75 | 6.75 | 6.75 |
| Potassium hydrogen carbonate | 0.00 | 20.00 | 40.00 | 50.00 |
| Hydroxypropyl cellulose | 3.00 | 3.00 | 3.00 | 3.00 |
| Low substitued hydroxypropyl cellulose | 25.00 | 25.00 | 25.00 | 25.00 |
| Microcrystalline cellulose | 51.00 | 32.00 | 10.00 | 0.00 |
| Magnesium stearate | 3.00 | 3.00 | 3.00 | 3.00 |
| Total weight of granules | 100.00 | 100.00 | 100.00 | 100.00 |
| Opadry yellow | 3.00 | 3.00 | 3.00 | 3.00 |
| Total weight of tablets | 103.00 | 103.00 | 103.00 | 103.00 |

**Table 4**

| Component | Example 5A | Example 6A | Example 7A | Example 8A |
|---|---|---|---|---|
| | mg/capsule | mg/capsule | mg/capsule | mg/capsule |
| Tablet | 103.00 (Ex.5) | 103.00 (Ex.6) | 103.00 (Ex.7) | 103.00 (Ex.8) |
| Hard gelatin capsule shell size 2 | 60.00 | 60.00 | 60.00 | 60.00 |
| Total weight of filled capsules | 163.00 | 163.00 | 163.00 | 163.00 |

### Examples 9-11: Formulations comprising meglumine as alkalizer

The formulations were prepared using wet granulation process, and then filled into capsules. The compositions of the formulations are shown in the below Table 5.

Wet granulation was performed with purified water as a solvent using a Lenvatinib mesylate, mannitol 25C (parlitol) (Mannitol, Roquette), meglumine (Merck), hydroxypropyl cellulose (HPGL, Nippon Soda), low-substituted hydroxypropyl cellulose (L-HPC (LH-11), Shin-Etsu Chemical), croscarmellose sodium (Mingtai Chemicals) and microcrystalline cellulose (FMC) according to the formulation proportions in Table 5. The granules of which a moisture content was reduced to be less than 2% by further drying were sized using a screen mill, so that their granule diameters were less than 1 mm. Then, microcrystalline cellulose and talc were added to the sized granules according to the formulation proportions in Table 5, and the mixture was thoroughly mixed using a diffusion mixer. Hard HPMC capsules size 4 were filled with 100 mg of the resultant granules to prepare capsules containing the Lenvatinib mesylate formulation.

**Table: 5**

| Component | Example 9 | Example 10 | Example 11 |
|---|---|---|---|
| | mg/capsule | mg/capsule | mg/capsule |
| Lenvatinib Mesylate | 12.25 | 12.25 | 12.25 |
| Mannitol 25 C | 9.75 | 9.75 | 9.75 |
| Meglumine | 20.00 | 40.00 | 40.00 |
| Hydroxy propyl cellulose | 3.00 | 3.00 | 3.00 |
| Low substitued Hydroxy propyl cellulose | 25.00 | 25.00 | -- |
| Croscarmellose sodium | -- | -- | 25.00 |
| Microcrystalline cellulose | 30.00 | 10.00 | 10.00 |
| Talc | 3.00 | 3.00 | 3.00 |
| Total weight of granules | 100.00 | 100.00 | 100.00 |
| HPMC Capsule shell size 4 | 38.00 | 38.00 | 38.00 |
| Total weight of filled capsules | 138.00 | 138.00 | 138.00 |

### Examples 12-14: Formulation comprising meglumine and alkali metal hydrogen carbonate as alkalizers

The formulations were prepared using wet granulation process, and then filled into capsules. The compositions of the formulations are shown in the below Table 6.

The wet granulation was performed with ethanol as a solvent using Lenvatinib mesylate, mannitol 25C (parlitol) (Mannitol, Roquette), Meglumine (Merck), potassium hydrogen carbonate (Merck), sodium hydrogen carbonate (sodium bicarbonate, Merck), hydroxypropyl cellulose (HPGL, Nippon Soda), low-substituted hydroxypropyl cellulose (L-HPC (LH-11), Shin-Etsu Chemical), croscarmellose sodium (Mingtai Chemicals) and microcrystalline cellulose (FMC) according to the formulation proportions in Table 6. The granules of which a moisture content was reduced to be less than 2% by further drying were sized using a screen mill, so that their granule diameters were less than 1 mm. Then, microcrystalline cellulose and talc were added to the sized granules according to the formulation proportions in Table 6, and the mixture was thoroughly mixed using a diffusion mixer. Hard HPMC capsules size 4 were filled with 100 mg of the resultant granules to prepare capsules containing the Lenvatinib mesylate.

**Table 6**

| Component | Example 12 | Example 13 | Example 14 |
|---|---|---|---|
| | mg/capsule | mg/capsule | mg/capsule |
| Lenvatinib Mesylate | 12.25 | 12.25 | 12.25 |
| Mannitol 25 C | 9.75 | 9.75 | 9.75 |
| Potassium hydrogen carbonate | 37.00 | - | 30.00 |
| Sodium hydrogen carbonate | | 20.00 | - |
| Meglumine | - | 17.00 | 7.00 |
| Hydroxypropyl cellulose | 3.00 | 3.00 | 3.00 |
| Low substitued hydroxypropyl cellulose | 25.00 | 25.00 | -- |
| Croscarmellose sodium | -- | -- | 25.00 |
| Microcrystalline cellulose | 10.00 | 10.00 | 10.00 |
| Talc | 3.00 | 3.00 | 3.00 |
| Total weight of granules | 100.00 | 100.00 | 100.00 |
| HPMC capsules size 4 | 38.00 | 38.00 | 38.00 |
| Total weight of filled capsules | 138.00 | 138.00 | 138.00 |

### Examples 15-18, 15A-18A: Formulation comprising different alkalizers

The formulations were prepared using wet granulation process, and then filled into HPMC capsules (Examples 15-18) or gelatine capsules (Examples 15A-18A). The compositions of the formulations are shown in the below Table 7.

The wet granulation was performed with purified water as a solvent using Lenvatinib mesylate, mannitol 25C (parlitol) (Mannitol, Roquette), polacrilin potassium (Purolite), magnesium oxide (Merck), magnesium aluminometasilicate (Fuji Chemical Industry), Syloid 244FP (Grace), hydroxypropyl cellulose (HPGL, Nippon Soda), low-substituted hydroxypropyl cellulose (L-HPC (LH-21), Shin-Etsu Chemical), microcrystalline cellulose (FMC) according to the formulation proportions in Table 7. The granules of which a moisture content was reduced to be less than 2% by further drying were sized using a screen mill, so that their granule diameters were less than 1 mm. Then, microcrystalline cellulose and talc were added to the sized granules according to the formulation proportions in Table 7, and the mixture was thoroughly mixed using a diffusion mixer. HPMC capsules size 4 (Table 7) or gelatin capsules size 4 (Table 8) were filled with 100 mg of the resultant granules to prepare capsules containing the Lenvatinib mesylate.

**Table 7**

| Component | Example 15 | Exapmple 16 | Example 17 | Example 18 |
|---|---|---|---|---|
| | mg/capsule | mg/capsule | mg/capsule | mg/capsule |
| Lenvatinib mesylate | 12.25 | 12.25 | 12.25 | 12.25 |
| Mannitol 25 C | 9.75 | 9.75 | 9.75 | 9.75 |
| Polacrilin potassium | 35.00 | -- | -- | -- |
| Magnesium aluminometasilicate (Neusiline US2) | -- | 35.00 | -- | -- |
| Syloid 244FP | -- | -- | 35.00 | -- |
| Magnesium Oxide | -- | -- | -- | 35.00 |
| Hydroxy propyl cellulose | 3.00 | 3.00 | 3.00 | 3.00 |
| Low substitued Hydroxy propyl cellulose | 25.00 | 25.00 | 25.00 | 25.00 |
| Microcrystalline cellulose | 12.00 | 12.00 | 12.00 | 12.00 |
| Talc | 3.00 | 3.00 | 3.00 | 3.00 |
| Total weight of granuless | 100.00 | 100.00 | 100.00 | 100.00 |
| HPMC capsules size 4 | 38.00 | 38.00 | 38.00 | 38.00 |
| Total weight of filled capsules | 138.00 | 138.00 | 138.00 | 138.00 |

**Table 8**

| Component | Example 15A | Example 16A | Example 17A | Example 18A |
|---|---|---|---|---|
| | mg/capsule | mg/capsule | mg/capsule | mg/capsule |
| Granules | 100.00 (Ex.15) | 100.00 (Ex.16) | 100.00 (Ex.17) | 100.00 (Ex.18) |
| Hard gelatin capsule shell size 4 | 38.00 | 38.00 | 38.00 | 38.00 |
| Total weight of filled capsules | 138.00 | 138.00 | 138.00 | 138.00 |

### Comparative example:

Table 9 shows formulations according to the present invention (as described in examples above) and formulations according to EP3384901.

**Table 9**

| Component | Present Invention | | According to EP3384901 (comparative) | |
|---|---|---|---|---|
| | Example 3 | Example 10 | | |
| | mg/capsule | mg/capsule | Ex. 1 | Ex 2 |
| Lenvatinib Mesylate | 12.25 | 12.25 | 12.25 | 12.25 |
| Sodium hydrogen carbonate | -- | -- | 33.00 | -- |
| Mannitol 25 C | 9.75 | 9.75 | 8.75 | 8.75 |
| Potassium hydrogen carbonate | 37.00 | -- | 15.00 | 33 |
| Meglumine | -- | 40.00 | -- | -- |
| Hydroxy propyl cellulose | 3.00 | 3.00 | -- | -- |
| Low substitued Hydroxy propyl cellulose | 25.00 | 25.00 | -- | -- |
| Microcrystalline cellulose | 10.00 | 10.00 | 43.00 | 43.00 |
| Talc | 3.00 | 3.00 | 3.00 | 3.00 |
| Total weight of granules | 100.00 | 100.00 | 100.00 | 100.00 |

### Dissolution tests

Dissolution tests were carried as per US FDA OGD recommended method:

| **Parameters** | **Dissolution Tester Conditions** |
|---|---|
| Medium | 0.1 N HCL, 900 mL |
| Apparatus | USP II (Paddle) + Sinker |
| Rotation | 50 rpm |
| Temperature | 37.0 ± 0.5°C |
| Sampling Time | 10, 15, 20, 30 mins |
| Sampling Volume | 10 mL |

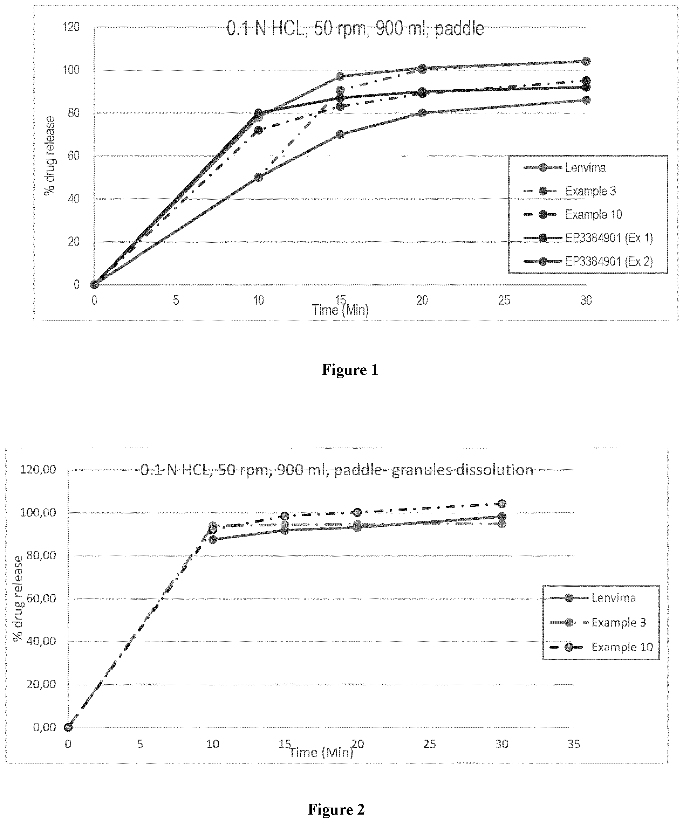

Figure 1 shows comparison of dissolution data for Example 3, Example 10 and two examples from EP 3384901 (see above the Comparative example for compositions).

Figure 1 shows that the formulation with sodium hydrogen carbonate of EP3384901 is slower in dissolution than Lenvima 10 mg (RLD sample) and also than the present invention with potassium hydrogen carbonate (Example 3). The formulation with potassium hydrogen carbonate of EP3384901 is slower in dissolution in comparison with both compositions of the present invention.

The formulations according to the invention show a comparative dissolution to that of Lenvima 10 mg (RLD sample), and the total dissolved amount is higher than for formulations of EP3384901.

Figure 2 shows a dissolution study performed on the open capsule, by performing dissolution on granules. Fig. 2 shows that the surface gelation issue has been resolved and test formulations shows similar drug release with marketed capsules formulation Lenvima 10 mg capsules from Eisai GmbH.

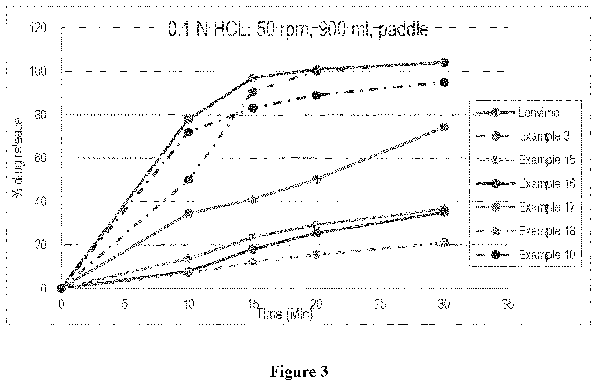

Figure 3 shows a dissolution study performed with formulations containing various alkalizers. The formulations were compared against the reference product available in the market is Lenvima 10 mg capsules from Eisai GmbH. Notably, the formulations of the present invention comprising potassium hydrogen carbonate and/or meglumine show the most similar drug release profile to the RLD product.

### Stability:

Based on the literature and QSAR report Lenvatinib formulation contains a mutagenic impurity.

Based on this mutagenicity prediction, and as per ICH M7 to be followed, considering the dosing duration as 1 to 10 years, the TTC (threshold for toxicological concern) limit will be 10 microgram/day and the control is 417 ppm. The same duration has been accepted by Reference product Lenvima 10 mg capsules by Eisai GmbH.

This impurity is a known human and animal metabolite identified as ME 104 in reference product literature. In monkeys it is reported as major metabolite in kidney (2.37%). Quantification data in human may be used later.

Freshly produced formulations were compared with formulations maintained for three months (3M) at 40°C and 75% relative humidity (RH), and at 25°C and 60% RH

| **Impurity Chemical name/ CAS no.** | **Initial data** | **3M 40°C/75%RH** | **3M 25°C/60%RH** |
|---|---|---|---|
| 4-(4-Amino-3-chlorophenoxy)-7-methoxy-6-quinolinecarboxamide/ CAS no. 417722-93-1 | Example 3: 195 ppm | Example 3: 340 ppm | Example 3: 230 ppm |
| | Example 10: 206 ppm | Example 10: 208 ppm | Example 10: 240 ppm |
| | | | |
| **Assay of Lenvatinib Mesylate (Each capsule contains 12.25 mg of Lenvatinib mesylate is equivalent to 10 mg Lenvatinib)** | Example 3: 101 % | Example 3: 103.5 % | NP |
| | Example 10: 102.6% | Example 10: 100.1% | |
| **Dissolution - 0.1 N HCL, 50 RPM, 900 ml, paddle (USP II apparatus)** | Example 3 | Example 3 | Example 3 |
| | Minimum- 90% | Minimum- 90% | Minimum- 86% |
| | Maximum - 101% | Maximum - 101% | Maximum - 98% |
| | Average - 92% | Average - 92% | Average - 91% |
| | RSD - 5.8% | RSD - 5.8% | RSD - 7.1% |
| | Example 10 | NP | NP |
| **Limit - Not less than 85 % in 20 mins** | | | |
| | Minimum- 82% | | |
| | Maximum - 90% | | |
| | Average - 89% | | |
| | RSD - 5.0% | | |

## Claims

1. Pharmaceutical formulation comprising Lenvatinib or a pharmaceutically acceptable salt thereof and at least one alkalizer.

2. Pharmaceutical formulation according to claim 1, further comprising at least one disintegrant.

3. Pharmaceutical formulation according to claim 1 or 2, wherein the weight ratio of Lenvatinib or a pharmaceutically acceptable salt thereof to the at least one alkalizer is from 1:0.3 to 1:6, preferably 1:1 to 1:4.

4. Pharmaceutical formulation according to claim 2, wherein the weight ratio of Lenvatinib or a pharmaceutically acceptable salt thereof to the at least one disintegrant is 1:1 to 1:6, preferably 1:1 to 1:3.

5. Pharmaceutical formulation according to claim 1, which contains from 10 to 20 wt. % of Lenvatinib or a pharmaceutically acceptable salt thereof, from 4 to 50 wt. % of at least one alkalizer, and optionally also 20 to 40 wt. % of at least one disintegrant.

6. Pharmaceutical formulation according to any one of claims 1 to 5, wherein the pharmaceutically acceptable salt of Lenvatinib is Lenvatinib mesylate.

7. Pharmaceutical formulation according to any one of claims 1 to 6, wherein the alkalizer is selected from the group consisting of magnesium oxide, calcium oxide, calcium chloride, sodium carbonate, lithium carbonate, potassium carbonate, sodium hydrogen carbonate, lithium hydrogen carbonate, potassium hydrogen carbonate, disodium hydrogenphosphate, sodium citrate, dipotassium hydrogenphosphate, sodium acetate, sodium lauryl sulphate, polacriline potassium, calcium hydroxide, sodium hydroxide, potassium hydroxide, meglumine, magnesium aluminometasilicate, mesoporous silica, colloidal silica and sodium hydroxide, with the proviso that sodium carbonate is only used in combination with at least one other alkalizer.

8. Pharmaceutical formulation according to any one of claims 1 to 6, wherein the alkalizer is selected from potassium hydrogen carbonate, potassium carbonate, meglumine, sodium carbonate, sodium hydrogen carbonate, polacrilin potassium, magnesium aluminometasilicate, mesoporous silica, colloidal silica and magnesium oxide, with the proviso that sodium carbonate is only used in combination with at least one other alkalizer.

9. Pharmaceutical formulation according to any one of claims 1 to 6, wherein the alkalizer is selected from potassium hydrogen carbonate, meglumine, mixture of potassium hydrogen carbonate with meglumine, and mixtures thereof with sodium carbonate.

10. Pharmaceutical formulation according to any one of claims 1 to 9, wherein the disintegrant is selected from corn starch, partially pregelatinized starch, hydroxypropyl starch, carmellose, carmellose sodium, carmellose calcium, carboxymethyl starch sodium, croscarmellose sodium, low-substituted hydroxypropylcellulose and crospovidone.

11. Pharmaceutical formulation according to any one of claims 1 to 9, wherein the disintegrant is selected from the group consisting of low-substituted hydroxypropyl cellulose and croscarmellose sodium.

12. Pharmaceutical formulation according to claim 1, which contains
- from 10 to 20 wt. % of Lenvatinib or Lenvatinib mesylate,
- from 4 to 50 wt. % of at least one alkalizer selected from potassium hydrogen carbonate, meglumine, mixture of potassium hydrogen carbonate with meglumine, and mixtures thereof with sodium carbonate, and
- 20 to 40 wt. % of at least one disintegrant selected from low-substituted hydroxypropyl cellulose and croscarmellose sodium.

13. Pharmaceutical formulation according to any one of claims 1 to 12, which is formulated into gelatine capsules, HPMC capsules, tablets or coated tablets.

14. Pharmaceutical formulation according to any one of claims 1 to 13 for use as a medicament.

15. Pharmaceutical formulation according to any one of claims 1 to 13 for use in the treatment of cancers, in particular thyroid cancers, renal cell carcinoma, hepatocellular carcinoma or endometrial carcinoma.
